# EUROPEAN PATENT APPLICATION

(11) **EP 3 662 815 A1**
(43) Date of publication of application: **10.06.2020**
(21) Application number: 18841295.1
(22) Date of filing: 17.07.2018
(51) Int. Cl.: A61B 5/00, G06F 13/00, G08C 15/00, G08C 15/06

(54) **SENSING DEVICE MANAGEMENT APPARATUS**

(30) Priority: 01.08.2017 JP 2017149189
(71) Applicant: Omron Corporation, Kyoto-shi, Kyoto 600-8530 (JP)
(72) Inventor: ODA, Toshihiko, Kyoto 619-0283 (JP); YAMATO, Tetsuji, Kyoto 619-0283 (JP); NAITO, Takeshi, Kyoto 619-0283 (JP); LEE, Sangryul, Kyoto 619-0283 (JP); YAMADA, Ryota, Kyoto 619-0283 (JP); MISUMI, Shuichi, Kyoto 619-0283 (JP)
(74) Representative: Hoffmann Eitle
(86) International application number: PCT/JP2018/026667
(87) International publication number: WO 2019/026602

(57) **Abstract**

A sensing device management apparatus easily and appropriately generates a data catalog corresponding to sensor metadata. A device information obtaining unit (11d) obtains, based on data obtained by a sensing device (2) for performing sensing on a measurement target, an address for obtaining device information about the sensing device (2), and obtains the device information by accessing the address. A data catalog generation unit (11e) generates a data catalog (100) using the device information about the sensing device (2) obtained by the device information obtaining unit (11d).

## Description

### FIELD

The present invention relates to a technique for distributing sensing data between a provider and a user.

### BACKGROUND

A sensor network system has been developed to distribute sensing data sensed by sensing devices between a provider and a user (refer to, for example, Patent Literature 1). Each sensing device herein is a sensor, or a device connectable to multiple sensors.

A provider registers, with a network server, a sensing device, and also sensor metadata about sensing data to be sensed and provided by the sensing device. A user registers, with the network server, an application that uses sensing data, and also application metadata about sensing data to be used by the application. The sensor metadata is information about a sensor, and also about the attributes of sensing data obtained by the sensor. The application metadata is information about an application, and also about the attributes of sensing data to be used by the application.

The network server performs matching using the sensor metadata and the application metadata, and retrieves a sensing device that provides sensing data that satisfies a request from the application. The network server transmits a data flow control command to a sensor management device that manages the retrieved sensing device. The data flow control command causes a data provider (sensing device) to distribute sensing data to a data user (application).

### CITATION LIST

### PATENT LITERATURE

Patent Literature 1: Japanese Patent No. 5445722

### SUMMARY

### TECHNICAL PROBLEM

However, generating sensor metadata is time-consuming for a provider. As described above, the sensor metadata is information about a sensor and about the attributes of sensing data obtained by the sensor. To generate sensor metadata, many providers examine, for example, the specifications of sensing data output from sensing devices. The providers then research any unknown part of the examined specifications for the sensing data.

As described above, such a time-consuming operation of generating sensor metadata may discourage a sensing device owner from registering sensor metadata with a network server. In other words, the time-consuming operation of generating sensor metadata may discourage a sensing device owner from providing sensing data. This may then disturb the promotion of sensing data distribution.

Inappropriate sensor metadata registered with a network server may disable appropriate distribution of sensing data between a provider and a user. For example, when registering sensor metadata, the provider may make an input error in manually inputting data, or may fail to input an appropriate term. Also, providers with different vocabulary levels may register metadata using various terms. The matching process can fail due to such varying terms that carry the same meaning.

One or more aspects of the present invention are directed to a technique for easily and appropriately generating a data catalog corresponding to sensor metadata.

### SOLUTION TO PROBLEM

In response to the above issue, a sensing device management apparatus according to one or more aspects of the present invention has the structure described below.

A device information obtaining unit obtains device information about a sensing device for performing sensing on a measurement target. The device information is information about the sensing device (e.g., a device model, a device identifier, and set values in the device). The device information includes information used for generating a data catalog. When, for example, the sensing device stores the device information, the device information obtaining unit may obtain the device information from the sensing device. When, for example, the sensing device stores an address at which the device information is available, the device information obtaining unit may obtain the address from the sensing device, and obtain the device information by accessing the address.

A data catalog generation unit generates a data catalog using the device information about the sensing device obtained by the device information obtaining unit.

The data catalog generated by the data catalog generation unit is transmitted to a management server for managing the distribution of the sensing data on a network to allow the sensing data to be distributed on the network.

In this manner, this structure generates the data catalog without a sensing device owner examining, for example, the specifications of the sensing data output from the sensing device. This structure thus allows the provider to generate the data catalog easily and appropriately, thus promoting the distribution of sensing data. The sensing device owner may access various databases connected through a network, such as the Internet, to generate the data catalog. The structure can reduce this operation of collecting information used for generating the data catalog, and reduces the communication traffic for collecting information used for generating the data catalog. Also, the user interface for generating the data catalog can use less program source (the sensing device management apparatus can save memory), thus reducing the hardware cost for the sensing device management apparatus.

The data catalog generation unit may receive an edit to the data catalog generated using the device information. This structure allows the sensing device owner to edit the data catalogue when generating the data catalog. The sensing device owner thus easily generates the date catalog as intended.

The data catalog generation unit may generate the data catalog by, for example, registering the device information into a template for the data catalog. In this case, the data catalog generation unit receives the above edit to the data catalog generated by registering the device information into the template for the data catalog.

The sensing device management apparatus may further include a device selection unit that receives selection of a sensing device. The device information obtaining unit may obtain device information about the sensing device selected with the device selection unit.

The sensing device management apparatus may further include a device registration unit that registers a sensing device. The device selection unit may allow selection of the sensing device registered by the device registration unit.

The sensing device management apparatus may further include a data catalog transmission unit that transmits the data catalog generated by the data catalog generation unit to a management server for managing sensing data trading on a network.

The sensing device management apparatus may further include an output control unit that outputs an item of sensing data allowed to be distributed in the data catalog generated by the data catalog generation unit, and does not output an item of sensing data unallowed to be distributed in the data catalog. The sensing data is obtained by the sensing device performing sensing on the measurement target. This structure prevents items of sensing data unintended for distribution on a network from leaking to a third party through the network.

### ADVANTAGEOUS EFFECTS

The above aspects enable easy and appropriate generation of a data catalog and promote distribution of sensing data.

### BRIEF DESCRIPTION OF THE DRAWINGS

Fig. 1 is a schematic diagram of a sensing data distribution system.
Fig. 2 is a diagram describing the details of a data catalog.
Fig. 3 is a block diagram of a gateway (GW) terminal showing its main components.
Fig. 4 is a block diagram of a sensing device showing its main components.
Fig. 5 is a flowchart showing a process for registering the sensing device performed by the GW terminal according to an embodiment.
Fig. 6 is a flowchart showing the operation of the sensing device according to the embodiment.
Fig. 7 is a flowchart showing a process for generating the data catalog performed by the GW according to the embodiment.
Figs. 8A and 8B are diagrams of example screens for editing the data catalog performed by the GW terminal.
Figs. 9A and 9B are diagrams of example screens for editing the data catalog performed by the GW terminal.
Fig. 10 is a flowchart showing a process for providing sensing data performed by the GW terminal according to the embodiment.
Fig. 11 is a schematic diagram of a sensing data distribution system according to another embodiment.
Fig. 12 is a flowchart showing a process for generating a data catalog performed by a GW terminal according to the other embodiment.
Fig. 13 is a flowchart showing the operation of a sensing device according to the other embodiment.

### DETAILED DESCRIPTION

One or more embodiments of the present invention will now be described.

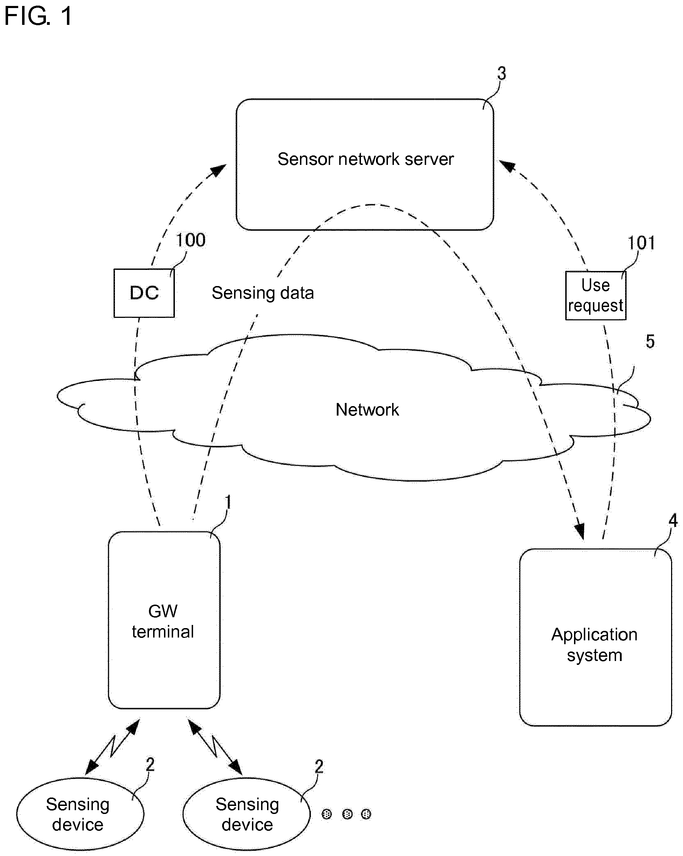

Fig. 1 is a schematic diagram of the sensing data distribution system according to an embodiment. The sensing data distribution system according to the embodiment includes a gateway (GW) terminal 1, sensing devices 2, a sensor network server 3, and an application system 4. The sensing data distribution system distributes sensing data traded between a provider and a user. The GW terminal 1 corresponds to a sensing device management apparatus in an aspect of the present invention. Each sensing device 2 is a sensor, or a device connectable to multiple sensors. The sensor network server 3 corresponds to a management server in an aspect of the present invention.

The GW terminal 1 and the sensing devices 2 are components of the provider providing sensing data. The application system 4 is a component of the user using sensing data. The sensor network server 3 defines a sensing data distribution market that serves as a marketplace for distributing sensing data on the Internet, or specifically a sensing data trading market (SDTM). The GW terminal 1, the sensor network server 3, and the application system 4 are connected through a network 5 to allow data communication between them. In the SDTM, the sensing data to be traded is obtained by the sensing devices 2 performing sensing on measurement targets.

The provider transmits a data catalog 100 (DC 100), which is associated with sensing data to be traded (to sell) in the SDTM, and registers the data catalog 100 with the sensor network server 3. The data catalog 100 stores attribute information about sensing data to be provided. The user transmits a use request 101, which is associated with sensing data to be traded (to purchase) in the SDTM to the sensor network server 3. The use request 101 includes attribute information about sensing data to be used. The sensor network server 3 performs matching for retrieving a provider that provides sensing data satisfying the use request 101 based on the registered data catalog 100 and the use request 101. The sensor network server 3 also performs a data flow control process for transmitting, to the extracted provider, a command (data flow control command) for transmitting the sensing data to the user. The provider transmits sensing data to the user in response to the data flow control command.

The provider may transmit the sensing data to the user through the sensor network server 3 in response to the data flow control command.

The sensor network server 3 is connectable to multiple provider systems (the GW terminal 1 and the sensing devices 2) with the network 5. The sensor network server 3 is connectable to multiple user application systems 4 with the network 5. Fig. 1 shows a single provider and a single user. Sensing data may be transmitted from the provider to the user through the sensor network server 3 as shown in Fig. 1, or may be transmitted from the provider to the user directly without using the sensor network server 3.

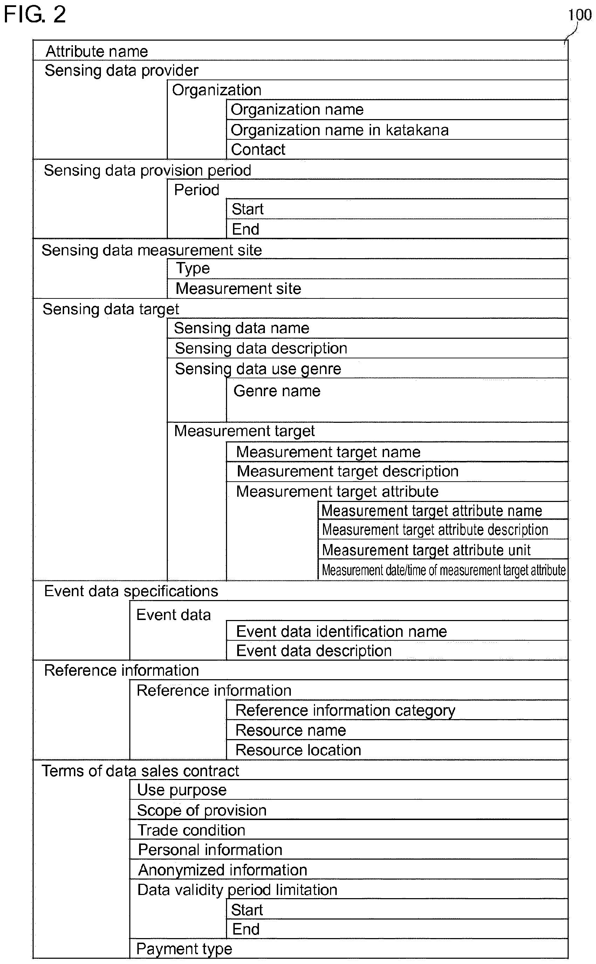

The data catalog 100 will now be described. Fig. 2 is a diagram describing the details of the data catalog 100. The data catalog 100 includes attribute information about the sensing data to be traded in the SDTM. As shown in Fig. 2, the data catalog 100 mainly includes a sensing data provider, a sensing data provision period, a sensing data measurement site, a sensing data target, event data specifications, reference information, and the terms of a data sales contract.

The attribute information about the sensing data provider is associated with an organization (individual or business entity) that provides sensing data, and includes the name of the organization (organization name), the name of the organization written in katakana characters (organization name in katakana), and the contact of the organization (contact).

The attribute information about the sensing data provision period is associated with a period for providing sensing data, and includes the start date of the sensing data provision (start) and the end date of the sensing data provision (end).

The attribute information about the sensing data measurement site is associated with a site at which the measurement target undergoes sensing, and includes the type and the measurement site. The type indicates either fixed, indicating that the measurement site is fixed at any measurement, or variable, indicating that the measurement site varies at every measurement. The measurement site is shown for the type indicating fixed.

The attribute information about the sensing data target is associated with sensing data, and includes the name of the sensing data (sensing data name), a brief description of the sensing data (sensing data description), a genre in which the sensing data is used (genre name), and the measurement target. The measurement target is shown for each measurement target, and includes information indicating the name, description, and attribute of the measurement target. The measurement target attribute includes information indicating the name, description, unit, and type of the measurement date and time (either intermittent or continuous) of the measurement target attribute.

The attribute information about the event data specifications includes a label name in an event condition (event data identification name) and the meaning and data representation of an event data value (event data description).

The attribute information about reference information includes a category indicating that the reference information is, for example, a specification, sample data, a product catalog, or a measurement example (reference information category), the document name of the reference information (resource name), and a uniform resource identifier (URI) at which the reference information is published (resource location).

The attribute information about the terms of the data sales contract is associated with the sensing data trading, and includes the use purpose of the sensing data (for profit, non-profit, or unlimited), the scope of provision indicating whether to provide the sensing data to a third party, trade conditions indicating, for example, whether sensing data is unduplicable, duplicable, modifiable, or processible, personal information indicating whether the sensing data includes personal information, anonymized information indicating whether the sensing data includes anonymized information, a data validity period limitation indicating the start date and the end date of the validity period of the sensing data, and the payment type indicating a method of payment of the sensing data fee.

As described above, the provider registers, with the sensor network server 3, the data catalog 100 of the sensing data to be traded in the SDTM. More specifically, the provider generates the data catalog 100 (shown in Fig. 2) of the sensing data to be traded in the SDTM. As described later, the sensing data distribution system according to the present embodiment enables the provider to generate the data catalog 100 shown in Fig. 2 appropriately and easily.

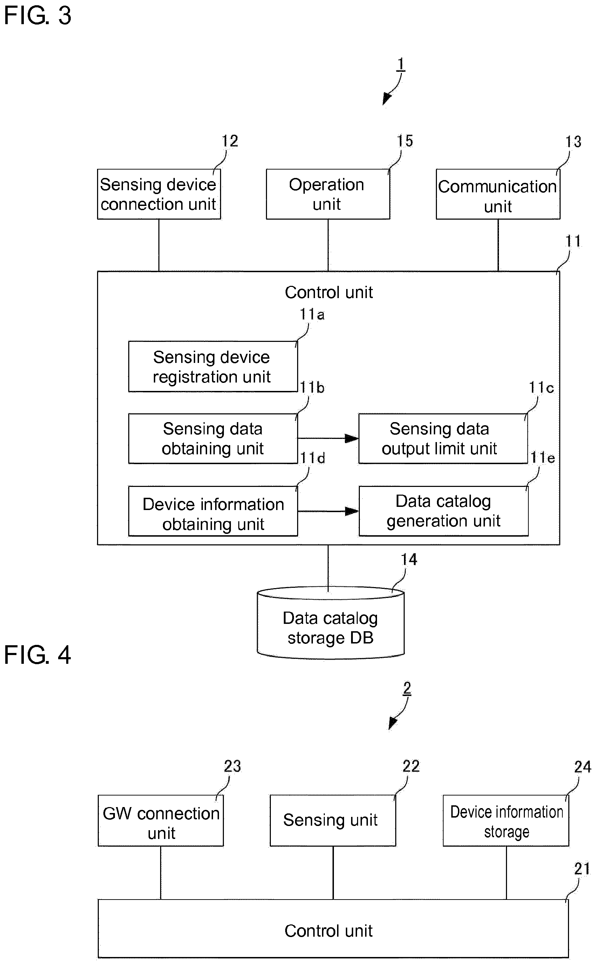

Fig. 3 is a block diagram of the GW terminal 1 showing its main components. The GW terminal 1 includes a control unit 11, a sensing device connection unit 12, a communication unit 13, a data catalog storage DB 14, and an operation unit 15. The GW terminal 1 may be a personal computer (PC), a mobile terminal such as a smartphone or a tablet, or may be any other information processing device. The GW terminal 1 is a smartphone in this embodiment.

The control unit 11 controls the operation of each main component of the GW terminal 1. The control unit 11 includes a sensing device registration unit 11a, a sensing data obtaining unit 11b, a sensing data output limit unit 11c, a device information obtaining unit 11d, and a data catalog generation unit 11e. These units 11a to 11e will be described in detail later.

The sensing device connection unit 12 connects the sensing devices 2 with wires or wirelessly. The sensing device connection unit 12 functions as an interface for controlling input and output of data with the sensing devices 2.

The communication unit 13 controls data communication with the sensor network server 3 and the application system 4 through the network 5.

The data catalog storage database (data catalog storage DB) 14 stores the data catalog 100. The data catalog storage DB 14 stores a template for the data catalog 100.

The operation unit 15 receives an input operation performed by an operator operating the GW terminal 1. The operation unit 15 includes a display and a touch panel attached to the screen of the display. The operation unit 15 also controls the screen display on the display. The operation unit 15 has the structure corresponding to a device selection unit in an aspect of the present invention.

Fig. 4 is a block diagram of a sensing device 2 showing its main components. The sensing device 2 includes a control unit 21, a sensing unit 22, a gateway connection unit (GW connection unit) 23, and a device information storage 24. The sensing device 2 may be an environmental sensor for sensing, for example, temperature, humidity, atmospheric pressure, noise, illuminance, and ultraviolet rays, a vital sign sensor for sensing, for example, a maximum blood pressure, a minimum blood pressure, a pulse, a body motion flag, and a weight, or a sensor other than these sensors. The sensing device 2 may perform sensing on a single measurement target or a plurality of measurement targets.

The control unit 21 controls the operation of each main component of the sensing device 2.

The sensing unit 22 includes a sensor for performing sensing on a measurement target. The sensing unit 22 may include a single sensor or a plurality of sensors. The sensing unit 22 corresponds to a sensor in an aspect of the present invention.

The GW connection unit 23 connects the GW terminal 1 with wires or wirelessly. The GW connection unit 23 functions as an interface for controlling input and output of data with the GW terminal 1. The GW connection unit 23 corresponds to a transmission unit in an aspect of the present invention.

The device information storage 24 stores device information. The device information is about a sensing device (e.g., a device model, a device identifier, and set values in a device). The device model indicates the model of a device. The device identifier is a code for identifying each device uniquely set at the shipment from the factory. The set values in a device are parameters regulating, for example, the operation of the device or signals to be input or output. The device information may include the measurement target attributes (the name, the description, the unit, and the measurement date and time of the measurement target attribute) stored in the data catalog 100 shown in Fig. 2 for each measurement target for the sensing unit 22. In place of or in addition to these items, the device information may include, for example, the reference information about the sensing device 2 (reference information category (for example, a manual, specifications, or an instruction of the sensing device 2), a resource name (e.g., XX manual, YY specification, or ZZ instruction), or a resource location (for example, http://www.example.com/XXspecification.pdf)), and whether the sensing data includes personal information or whether the sensing data includes the anonymized information. The device information storage 24 corresponds to a storage in an aspect of the present invention.

Referring back to Fig. 3, the units 11a to 11 e included in the control unit 11 in the GW terminal 1 will now be described. The sensing device registration unit 11a registers the device identifier of the sensing device 2 in the GW terminal 1. The GW terminal 1 can register multiple sensing devices 2. The GW terminal 1 cannot be used to trade, in the SDTM, sensing data sensed by an unregistered sensing device 2. In other words, sensing data sensed only by sensing devices 2 registered with the GW terminal 1 is available for trading in the SDTM. The sensing device registration unit 11a corresponds to a device registration unit in an aspect of the present invention.

The provider can choose not to trade sensing data sensed by a sensing device 2 registered with the GW terminal 1 in the SDTM.

The sensing data obtaining unit 11b obtains sensing data sensed by a sensing device 2 from the sensing device 2 connected through the sensing device connection unit 12.

The sensing data output limit unit 11c filters and classifies sensing data sensed by the sensing device 2 and obtained by the sensing data obtaining unit 11b. Through filtering, the sensing data is classified into data items allowed to be transmitted to an external device (user) through the communication unit 13, and data items unallowed to be transmitted to the external device. The sensing data output limit unit 11c corresponds to an output control unit in an aspect of the present invention.

The device information obtaining unit 11d obtains, from a sensing device 2 connected to the sensing device connection unit 12, the device information stored in the device information storage 24 included in the sensing device 2. The device information obtaining unit 11d corresponds to a device information obtaining unit in an aspect of the present invention.

The data catalog generation unit 11e generates the data catalog 100 shown in Fig. 2 using the device information obtained by the device information obtaining unit 11d from the sensing device 2 and the template for the data catalog 100. The data catalog generation unit 11e receives an editing operation performed by the provider when generating the data catalog 100. The data catalog generation unit 11e corresponds to a data catalog generation unit in an aspect of the present invention.

The control unit 11 in the GW terminal 1 includes a hardware central processing unit (CPU), a memory, and other electronic circuits. The hardware CPU functions as the sensing device registration unit 11a, the sensing data obtaining unit 11b, the sensing data output limit unit 11c, the device information obtaining unit 11d, and the data catalog generation unit 11e described above. The memory has an area for expanding a sensing data catalog generation program according to one or more embodiments of the present invention and an area for temporarily storing data generated by executing the sensing data provision program. The control unit 11 may be a large scale integrated circuit (LSI) integrating, for example, a hardware CPU and a memory.

Although not specifically shown in detail, the sensor network server 3 includes a matching unit and a data flow control unit. The matching unit performs matching to extract a provider that provides sensing data satisfying the use request 101 based on the registered data catalog 100 and the use request 101. The data flow control unit performs a data flow control process to transmit, to the extracted provider, a command (data flow control command) for transmitting the sensing data to the user.

Although not specifically shown in detail, the application system 4 includes, for example, a server, a PC, and a mobile terminal. The application system 4 executes an application program using sensing data, and outputs the execution result. The application system 4 obtains the sensing data to be used for executing the application program from the provider.

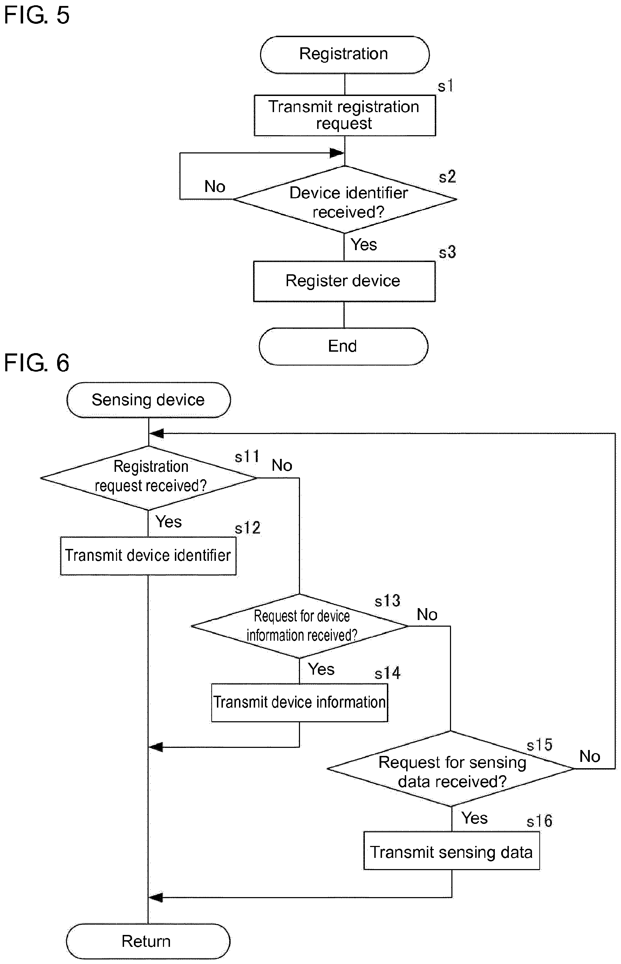

The processes performed by the GW terminal 1 for registering a sensing device 2, generating the data catalog 100, and providing the sensing data will now be described below. Fig. 5 is a flowchart showing a process for registering the sensing device 2 performed by the GW terminal 1 according to the embodiment. Fig. 6 is a flowchart showing the operation of a sensing device 2.

The sensing device registration unit 11a in the GW terminal 1 transmits a registration request to the sensing device 2 connected to the sensing device connection unit 12 (s1).

When the GW connection unit 23 receives the registration request transmitted from the GW terminal 1 (s11), the sensing device 2 transmits its device identifier to the GW terminal 1 (s12), and returns to s11. The sensing device 2 stores the device identifier into the device information storage 24.

In the GW terminal 1, when the sensing device connection unit 12 receives the device identifier transmitted from the sensing device 2 (s2), the sensing device registration unit 11a performs device registration to store the received device identifier in a memory (s3), and ends the processing.

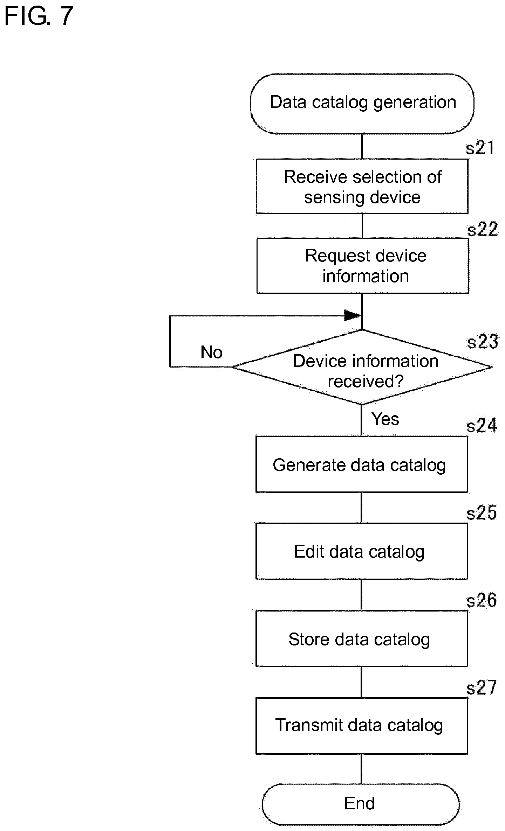

A process performed by the GW terminal 1 for generating the data catalog 100 will now be described. Fig. 7 is a flowchart showing the process for generating the data catalog 100 performed by the GW terminal 1 according to this embodiment. The GW terminal 1 receives selection of a sensing device 2 for which the data catalog 100 is to be generated (s21). The provider operates the operation unit 15 of the GW terminal 1, and selects a sensing device 2 for which the data catalog 100 is to be generated. For example, the GW terminal 1 shows, on the display, the device identifiers of the registered sensing devices 2. The provider selects a sensing device 2 for which the data catalog 100 is to be generated from the sensing devices 2 with the device identifiers on the display.

The GW terminal 1 may continuously perform the processing in s22 and subsequent steps described below using the sensing device 2 registered in s3.

The device information obtaining unit 11d in the GW terminal 1 requests device information from the sensing device 2 selected in s21 (s22). In this state, the GW terminal 1 performs error handling when the sensing device 2 selected in s21 is disconnected from the sensing device connection unit 12.

When the GW connection unit 23 receives the request for the device information transmitted from the GW terminal 1 (s13), the sensing device 2 transmits the device information stored in the device information storage 24 to the GW terminal 1 (s14), and returns to s11.

When the sensing device connection unit 12 receives the device information transmitted from the sensing device 2 (s23), the GW terminal 1 generates the data catalog 100 (s24). In s24, the data catalog generation unit 11e generates the data catalog 100 by registering the device information obtained by the device information obtaining unit 11d (device information received in s23) into the template for the data catalog 100 stored in the data catalog storage DB 14.

The data catalog 100 generated in s24 may include items without registered information. When the GW terminal 1 stores the information about the items (organization name, organization name in katakana, and contact) of the attribute information about the sensing data provider, the data catalog generation unit 11e may register the information with the data catalog 100.

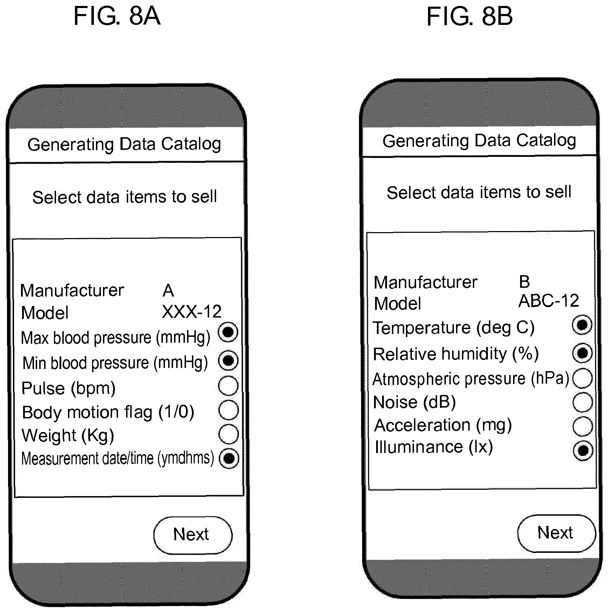

The data catalog generation unit 11e receives an edit to the data catalog 100 generated in s24 and generates the data catalog 100 (s25). In the GW terminal 1, the operation unit 15 receives an edit associated with selection of sensing data items to sell in the SDTM in s25. When, for example, the sensing device 2 is a blood pressure meter and outputs six items including a maximum blood pressure, a minimum blood pressure, a pulse, a body motion flag, a weight, and a measurement date and time as sensing data, the GW terminal 1 receives selection to sell or not to sell each item in the SDTM (refer to Fig. 8A). For example, when the sensing device 2 is an environmental sensor that outputs six sensing data items including temperature, relative humidity, atmospheric pressure, noise, acceleration, and illuminance, the GW terminal 1 receives, for each of these items, selection to sell or not to sell each item in the SDTM (refer to Fig. 8B). For the items with a check on their right in Figs. 8A and 8B (maximum blood pressure, minimum blood pressure, and measurement date and time in Fig. 8A and temperature, relative humidity, and illuminance in Fig. 8B) are selected to sell in the SDTM.

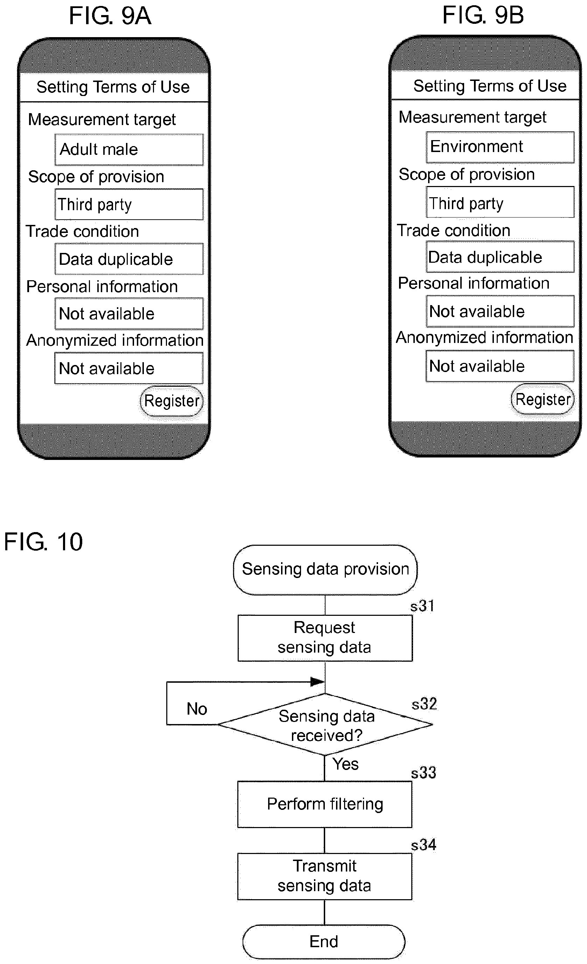

The operation unit 15 in the GW terminal 1 receives an edit to each item (including items not described at this time) in the data catalog 100 (refer to Figs. 9A and 9B). Figs. 9A and 9B are diagrams of example screens on the GW terminal 1 receiving an edit to a measurement target, the scope of provision, a trade condition, personal information, and anonymized information. In s25, the GW terminal 1 also receives an edit to other attributes not shown in Figs. 9A and 9B.

After the edit to the data catalog 100 is complete in s25, the GW terminal 1 registers the data catalog 100 generated this time into the data catalog storage DB 14, and the communication unit 13 transmits the generated data catalog 100 with the sensor network server 3 (s26 and s27). The sensor network server 3 registers the data catalog 100 transmitted from the GW terminal 1. The processing in s26 and the processing in s27 may be performed in either order.

With the GW terminal 1 according to the present embodiment, the provider may simply edit the data catalog 100 generated using the device information stored in the sensing device 2. The data catalog 100 generated by the GW terminal 1 in s24 is a draft data catalog for the provider. The provider edits the draft data catalog. This structure thus allows the provider to generate the data catalog 100 easily and appropriately, thus promoting the distribution of sensing data.

Although the GW terminal 1 performs the device registration by storing the device identifier transmitted from the sensing device 2 into the memory in s3 as described above, the GW terminal 1 may perform the device registration by storing the device information transmitted from the sensing device 2 into the memory. In this case, the sensing device 2 may transmit its device information in s12. This structure eliminates the processing in s22 and s23 performed by the GW terminal 1 and the processing in s13 and s14 performed by the sensing device 2. Further, the GW terminal 1 may generate the data catalog 100 in s24 (eliminate the editing operation performed by the provider).

The GW terminal 1 may perform the processing shown in Figs. 5 and 7 when detecting the sensing device connection unit 12 connected to the sensing device 2.

The GW terminal 1 also performs the sensing data provision shown in Fig. 10. The sensing data obtaining unit 11b in the GW terminal 1 requests sensing data from a sensing device 2 (s31). The sensing device 2 is connected to the sensing device connection unit 12 for obtaining the sensing data requested by the data flow control command transmitted by the sensor network server 3.

When receiving the request for the sensing data from the GW terminal 1 (s15), the sensing device 2 performs sensing on a measurement target with the sensing unit 22 and transmits the sensing data to the GW terminal 1 (s16).

The GW terminal 1 waits for the sensing device connection unit 12 to receive the sensing data transmitted from the sensing device 2 from which the GW terminal 1 has requested the sensing data (s32). When the sensing data obtaining unit 11b obtains the sensing data transmitted from the sensing device 2, the sensing data output limit unit 11c performs filtering (s33). In s33, the sensing data output limit unit 11c extracts the items of sensing data selected to sell in the SDTM in the data catalog 100 registered with the data catalog storage DB 14. In other words, in s33, the sensing data output limit unit 11c excludes the items of sensing data unselected to sell in the SDTM in the data catalog 100 registered with the data catalog storage DB 14.

The sensing device 2 may perform sensing on a measurement target in a timely manner and transmit the sensing data to the GW terminal 1 instead of performing the processing in s15 and s16. In this case, the GW terminal 1 may include, for example, a memory for storing the sensing data transmitted from the sensing device 2 for a predetermined period. The GW terminal 1 may perform filtering in s33 on the sensing data stored in the memory and transmitted from the sensing device 2 instead of performing the processing in s31 and s32.

The communication unit 13 in the GW terminal 1 outputs the sensing data filtered in s33 to the application system 4 (s34), and ends the process. The sensing data may be transmitted to the application system 4 through the sensor network server 3 or to the application system 4 directly from the GW terminal 1.

In this manner, the GW terminal 1 according to the present embodiment does not output, to external devices, items of sensing data unselected to sell in the SDTM in the data catalog 100 registered with the data catalog storage DB 14. This prevents the items of sensing data unselected by the provider to sell in the SDTM from leaking to a third party.

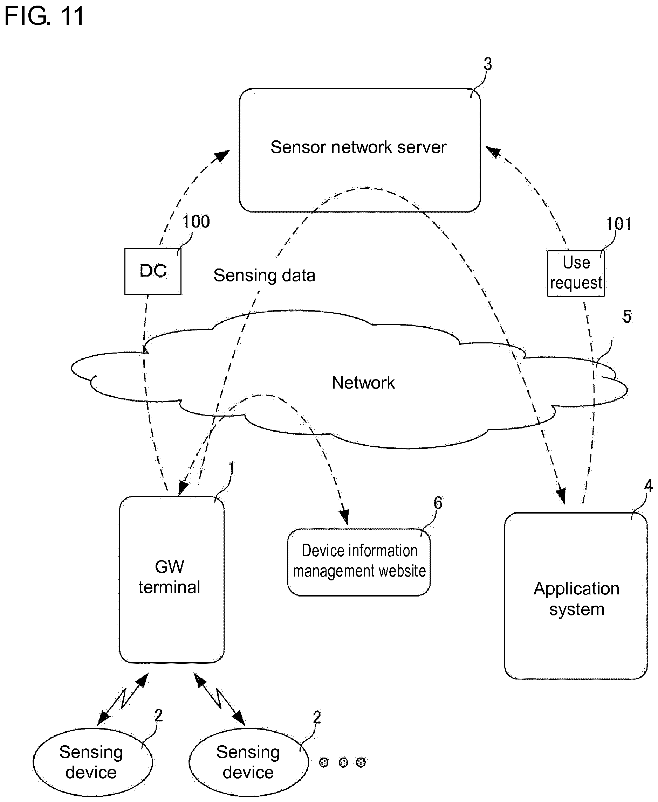

A sensing data distribution system according to another embodiment will now be described. Fig. 11 is a schematic diagram of the sensing data distribution system according to the other embodiment. The sensing data distribution system according to the embodiment differs from the sensing data distribution system according to the above embodiment in that the device information storage 24 in each sensing device 2 stores an address of a website storing the device information instead of storing the device information. In the example shown in Fig. 11, a device information management website 6 stores the device information about the sensing device 2.

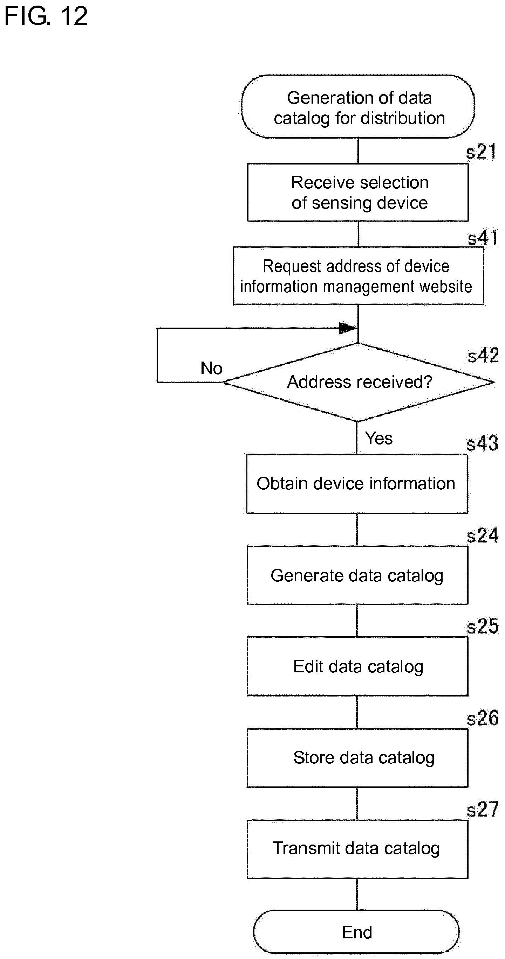

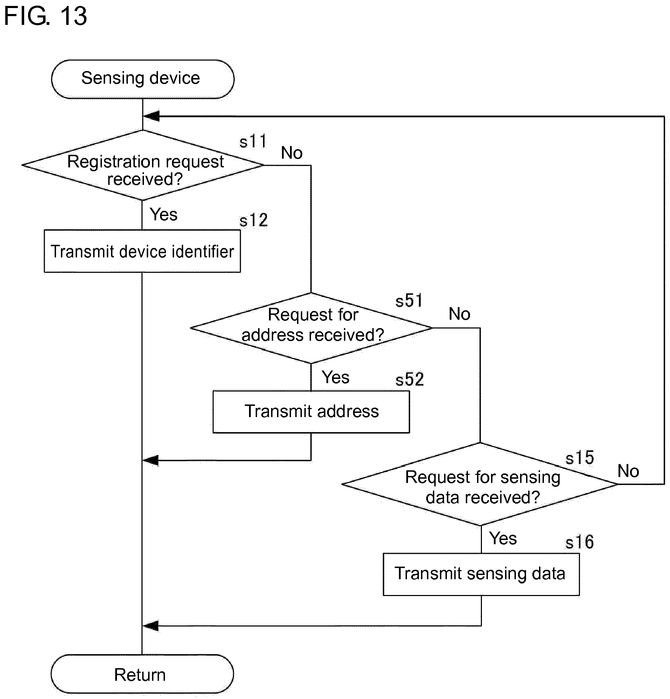

In the sensing data distribution system according to this embodiment, the GW terminal 1 performs the registration shown in Fig. 5 and the sensing data provision shown in Fig. 10. The GW terminal 1 also performs data catalog generation shown in Fig. 12. The sensing device 2 performs the processing shown in Fig. 13. In Fig. 12, the same processing steps as in Fig. 7 are given the same step numerals. In Fig. 13, the same processing steps as in Fig. 6 are given the same step numerals.

When receiving selection of a sensing device 2 in s21, the GW terminal 1 requests an address of the device information management website 6 from the selected sensing device 2 (s41). In this state, the GW terminal 1 performs error handling when the sensing device 2 selected in s21 is disconnected from the sensing device connection unit 12.

When the GW connection unit 23 receives the request for the address of the device information management website 6 transmitted from the GW terminal 1 (s51), the sensing device 2 transmits the address of the device information management website 6 stored in the device information storage 24 to the GW terminal 1 (s52), and returns to s11.

When the sensing device connection unit 12 receives the address of the device information management website 6 transmitted from the sensing device 2 (s42), the GW terminal 1 obtains the device information about the sensing device 2 (s43). In s43, the GW terminal 1 accesses the address of the device information management website 6 transmitted from the sensing device 2 and obtains the device information about the sensing device 2. After obtaining the device information about the sensing device 2 in s43, the GW terminal 1 performs the processing in s24 to s27 described above.

In this manner, the sensing data distribution system according to this embodiment differs from the embodiment described above in that the device information about the sensing device 2 is obtained from the device information management website 6 rather than from the sensing device 2. In the above example, the GW terminal 1 obtains the address of the device information management website 6, at which the device information is available, directly from the sensing device 2. However, the GW terminal 1 may instead access a server on the network 5 where key information that is other information obtained by the sensing device 2, such as a device model or a device identifier, is convertible into an address for obtaining the device information, and obtain the address.

With the GW terminal 1 according to the present embodiment as well, the provider may simply edit the data catalog 100 generated using the device information stored in the sensing device 2. This structure thus allows the provider to generate the data catalog 100 easily and appropriately, thus promoting the distribution of sensing data.

The present invention is not limited to the above embodiments, but the components may be modified without departing from the spirit and scope of the invention in its implementation. The components described in the above embodiments may be combined as appropriate to provide various aspects of the invention. For example, some of the components described in the above embodiments may be eliminated. Further, components in different embodiments may be combined as appropriate.

The above embodiments may be partially or entirely expressed in, but not limited to, the following forms shown in the appendixes below.

### Appendix 1

A sensing device management apparatus, comprising:
at least one hardware processor configured to
obtain device information about a sensing device for performing sensing on a measurement target; and
generate a data catalog using the obtained device information about the sensing device.

### Appendix 2

A data catalog generation method implementable by at least one hardware processor, the method comprising:
obtaining device information about a sensing device for performing sensing on a measurement target; and
generating a data catalog using the obtained device information about the sensing device.

### REFERENCE SIGNS LIST

- 1: gateway terminal (GW terminal)
- 2: sensing device
- 3: sensor network server
- 4: application system
- 5: network
- 6: device information management website
- 11: control unit
- 11a: sensing device registration unit
- 11b: sensing data obtaining unit
- 11c: sensing data output limit unit
- 11d: device information obtaining unit
- 11e: data catalog generation unit
- 12: sensing device connection unit
- 13: communication unit
- 14: data catalog storage database (data catalog storage DB)
- 15: operation unit
- 21: control unit
- 22: sensing unit
- 23: gateway connection unit (GW connection unit)
- 24: device information storage
- 100: data catalog (DC)
- 101: use request

## Claims

1. A sensing device management apparatus (1), comprising:
a device information obtaining unit (11d) configured to obtain, based on data obtained by a sensing device (2) for performing sensing on a measurement target, an address for obtaining device information about the sensing device (2), and obtain the device information by accessing the address; and
a data catalog generation unit (11e) configured to generate a data catalog (100) using the device information about the sensing device (2) obtained by the device information obtaining unit (11d).

2. The sensing device management apparatus (1) according to claim 1, further comprising:
a data catalog transmission unit (13) configured to transmit the data catalog (100) generated by the data catalog generation unit (11e) to a management server (3) for managing sensing data trading on a network (5).

3. The sensing device management apparatus (1) according to claim 1 or claim 2, further comprising:
an output control unit (11c) configured to output an item of sensing data allowed to be distributed in the data catalog (100) generated by the data catalog generation unit (11 e), and not to output an item of sensing data unallowed to be distributed in the data catalog (100), the sensing data being obtained by the sensing device (2) performing sensing on the measurement target.

4. The sensing device management apparatus (1) according to any one of claims 1 to 3, wherein
the data catalog generation unit (11e) receives an edit to the data catalog (100) generated using the device information.

5. The sensing device management apparatus (1) according to any one of claims 1 to 4, wherein
the data catalog generation unit (11e) generates the data catalog (100) by registering the device information into a template for the data catalog (100).

6. The sensing device management apparatus (1) according to any one of claims 1 to 5, further comprising:
a device selection unit (15) configured to receive selection of a sensing device (2),
wherein the device information obtaining unit (11d) obtains device information about the sensing device (2) selected with the device selection unit (15).

7. The sensing device management apparatus (1) according to claim 6, further comprising:
a device registration unit (11a) configured to register a sensing device (2),
wherein the device selection unit (15) selects the sensing device (2) registered by the device registration unit (11 a).

8. A sensing device (2), comprising:
a sensor (22) configured to perform sensing on a measurement target;
a storage (24) storing an address for obtaining device information to be used for generating a data catalog (100) of sensing data obtained by the sensor (22); and
a transmission unit (23) configured to transmit, in response to a request from a sensing device management apparatus (1), the address for obtaining the device information stored in the storage (24) to the sensing device management apparatus (1).

9. A sensing data distribution system, comprising:
the sensing device management apparatus (1) according to any one of claims 1 to 7; and
the sensing device (2) according to claim 8.

10. A data catalog generation method implementable by a computer, the method comprising:
obtaining, based on data obtained by a sensing device (2) for performing sensing on a measurement target, an address for obtaining device information about the sensing device (2), and obtaining the device information by accessing the address; and
generating a data catalog (100) using the obtained device information about the sensing device (2).

11. A data catalog generation program causing a computer to implement:
obtaining, based on data obtained by a sensing device (2) for performing sensing on a measurement target, an address for obtaining device information about the sensing device (2), and obtaining the device information by accessing the address; and
generating a data catalog (100) using the obtained device information about the sensing device (2).
